# EUROPEAN PATENT SPECIFICATION

(11) **EP 2 435 021 B1**
(45) Date of publication and mention of the grant of the patent: **03.07.2013**
(21) Application number: 10727845.9
(22) Date of filing: 25.05.2010
(51) Int. Cl.: A61K 33/08, A61K 9/00, A61K 45/06, A61K 31/728, A61K 31/737

(54) **USE OF A GLYCOSAMINOGLYCAN FIXED COMBINATION AND CHEWABLE COMPOSITION COMPRISING SAID FIXED COMBINATION**
VERWENDUNG EINER FIXEN GLYCOSAMINOGLYCAN-KOMBINATION UND EINER DIESE FIXE KOMBINATION UMFASSENDEN KAUBAREN ZUSAMMENSETZUNG
UTILISATION D'UNE COMBINAISON FIXE COMPRENANT DU GLYCOSAMINOGLYCAN ET COMPOSITION MACHABLE COMPRENANT LADITE COMBINAISON

(30) Priority: 25.05.2009 EP 09006955; 13.11.2009 EP 09014212
(43) Date of publication of application: 04.04.2012
(73) Proprietor: PHARCOTERM S.R.L., 20095 Cusano Milanino (MI) (IT)
(72) Inventor: PIZZONI, Angelo, I-20095 Cusano Milanino (IT); PALMIERI, Beniamino, 41125 Modena (IT)
(74) Representative: Trupiano, Federica
(86) International application number: PCT/IB2010/001227
(87) International publication number: WO 2010/136872

(56) References cited:
- WO-A-02/09637
- WO-A-89/07932
- WO-A-2006/049463
- US-A- 3 452 138
- US-A1- 2002 068 718
- DATABASE WPI Week 198616 Thomson Scientific, London, GB; AN 1986-103236 XP002550286, -& JP 61 047418 A (SEIKAGAKU KOGYO CO LTD) 7 March 1986 (1986-03-07) cited in the application

## Description

### SUMMARY OF THE INVENTION

The present invention concerns the use of a fixed combination of hyaluronic acid, chondroitin sulfate and aluminum hydroxide for the preparation of medicaments for treating gastric disorders, in particular for the prevention or for the treatment of gastritis or of gastric or duodenal endothelial damages, such as erosions or ulcers and a chewable glycosaminoglycan composition comprising said fixed combination for the prevention or for the treatment of gastritis or of gastric or duodenal endothelial damages, such as erosions or ulcers.

### BACKGROUND OF THE INVENTION

Glycosaminoglycans are a family of polysaccharides formed by the repetition of a uronic acid, glucuronic or iduronic acid, α 1→4 or β 1→3 linked to a hexosamine residue, glucosamine or galactosamine.

Hyaluronic acid is a glycosaminoglycan natural product which occurs as a regular, unsulfated macromolecule formed by a linear, repetitive disaccharide sequence of glucuronic acid β 1→3 linked to N-acetylglucosamine.

Chondroitin sulfate also is a glycosaminoglycan natural product formed by the repetition of glucuronic acid, β 1→3 linked to a 4- and/or 6-O-sulfated N-acetylgalactosamine. It is mainly present in two distinct forms chondroitin-4-sulfate (or ChSA) and chondroitin-6-sulfate (or ChSC) and is located in cartilages and in epithelia like gastric mucosa or urethelium. In the pathologies in which the amount of chondroitin sulfate is low, such as gastritis or interstitial cystitis, the administration of chondroitin sulfate helps to mitigate the inflammation and the correlate damages due to the low chondroitin sulfate content.

Unless otherwise specified, in the present description the terms "hyaluronic acid" and "chondroitin sulfate" designate hyaluronic acid and chondroitin sulfate in sodium salt form, the dosages given herein below being referred to sodium hyaluronate and sodium chondroitin sulfate.

### PRIOR ART

EP 136782 discloses a combination of hyaluronic acid, or of the sodium, potassium, magnesium and calcium salt thereof, and chondroitin sulfate, or the sodium, potassium, magnesium or calcium salt thereof, used for the treatment of interstitial cystitis This document generally mentions combinations in an aqueous buffer at pH of 7 to 8 wherein the concentration of the active components is of from 0.1 to 10% but specifically discloses compositions in which 5.3 percent by weight of the total composition is chondroitin sulfate and 4.2 percent is hyaluronate.

US 2006234978 describes the use of a solution containing both hyaluronic acid and chondroitin sulfate (optionally also with glucosamine) to treat the interstitial cystitis. The amounts of chondroitin sulfate and of hyaluronic acid disclosed therein are 0.5-1.5 g and 10-50 mg, respectively, per dosage unit.

US 2002/0068718, become US 6,924,273 (see also US 2005/0182022), discloses chondroprotective/restorative compositions, to be administered especially as animal feed to racing thoroughbreds, comprising hyaluronic acid, alone or in combination with chondroitin sulfate and/or glucosamine, together with a gelling agent such as a cellulose derivative. In particular, the document describes
(a) a composition comprising glucosamine sulfate, hyaluronic acid and an optional pharmaceutical carrier;
(b) a composition comprising chondroitin sulfate, hyaluronic acid and an optional pharmaceutical carrier;
(c) a composition comprising glucosamine sulfate, chondroitin sulfate, hyaluronic acid and an optional pharmaceutical carrier;
(d) a composition comprising a nutritionally effective feed base and hyaluronic acid;
(e) a composition comprising hyaluronic acid, a therapeutic drug and an optional pharmaceutical carrier; and
(f) a composition in gel form comprising hyaluronic acid and a sufficient amount of carboxymethylcellulose to make a paste,
the therapeutic drug being also referred to as as "bio-effective or drug component" or "bio-effecting agent". Said bio-effective or drug component is selected from the group consisting classes of therapeutic agents not including agents combating gastric disorders. In a list of specific bio-effecting agents, this document mentions aluminum acetate, aluminum carbonate, aluminum chlorhydrate and aluminum hydroxide. In particular, the document describes a gel composition comprising 1% wt. of sodium hyaluronate, 4% wt. of condroitin sulfate and 1% sodium carboxymethyl cellulose.

The Japanese patent application published as JP 61-047418 discloses a preparation having protecting action on gastric mucosa, lowering lipid in blood, and improving action on constipation, comprising chondroitin sulfuric acid or its salts (1%-40% by weight), hyaluronic acid and its salts (0.01%-5% by weight), lecithin (0.5%-20% by weight), and protein(35%-98.5% by weight) to be administered in form of solution, granules, tablets, pastilles without mentioning chewable tablets. This document specifically states that, beside the chondroitin sulfate, hyaluronic acid, lecithin and protein active ingredients, vegetable extracts, vitamin A, B1, B6, B12, C, D, E and panthotenic acid, nicotinamide, biotine, pteroylglutamic acid, inositol, taurine, Ginseng extract or various amino acids and various minerals may be added, the total proportion thereof being less than 10% in respect of the total weight of the four active ingredients. No particular mineral is mentioned in this document.

WO 00/67799 (see also EP 1173218, EP 1614431 and US 6,610,667) discloses a composition for the prevention or treatment of esophagus disorders caused by reflux, comprising an alginate and two types of gums (xantan/carrageenan and galactomannan/glucomannan gums). This composition has high bioadhesive properties and is capable of detachably adhering to desired regions of the esophagus, thus providing a protective and a direct healing effect. However, the disclosed compositions are very viscous because, even in the case of liquid preparations, the viscosity ranges from 500 to 10,000 mPa.

WO 2008/157775 discloses a composition, and a method of use thereof, for treating connective tissue damage in man and in animals, which comprises a therapeutically effective amount of chondroitin sulfate, N-acetyl D-glucosamine, and hyaluronan (hyaluronic acid) for treating connective tissue damage such as arthritic disease, osteoarthritis, rheumatoid arthritis, osterochondrosis dessicans, cartilage damage, joint injury, joint inflammation, joint synovitis, degenerative joint disease, fracture, tendon damage, ligament damage, skeletal damage, musculoskeletal damage, fiber damage, adipose tissue damage, blood cell damage, and plasma damage.

US 3, 452,138 discloses chewable tablets comprising urea and antacids such as aluminium hydroxide, alone or in combination with other antacids such as magnesium hydroxide, magnesium oxide, magnesium carbonate and magnesium trisilicate as effective medication for the control of gastric hyperacidity and gastric ulcers.

It is also known that chondroitin sulfate is an effective inhibitor of pepsin induced damage of the gastroduodenal mucosa having gastroduodenal ulcer healing properties (see for example Crandall L.A et al., Proc. Soc Exp. Biol. Med. 30, 704-708,1933) and that a combination of chondroitin sulfate and aluminum hydroxide was also marketed (CONDROVIS™) but retired from the market. In fact, it is also known that compositions of chondroitin sulfate and aluminum hydroxide, as antacid and gastric mucosa protective agents, generally have a palliative effect and involve very long treatments in the case of the presence of a gastroduodenal ulcers, without being able to heal them.

### SUMMARY OF THE INVENTION

It has been found that a fixed combination of hyaluronic acid, chondroitin sulfate and aluminum hydroxyde may be used for the prevention or for the treatment of gastritis or of gastric or duodenal endothelial damages, such as erosions or ulcers, including gastritis or gastric endothelial damages, including those caused by adverse effects of drugs. Hyaluronic acid is capable of healing a damaged gastric wall. In addition, chondroitin sulfate selectively fixes itself to the gastric mucosa and forms a protective layer which is not attached by the gastric enzymes. Aluminum hydroxide, acting as an antacid agent, hinders the gastric erosion. Thus, the fixed combination of the invention is not absorbed but adheres to the gastric mucosa and protects the gastro-duodenal wall from the attack of endogenous acidic production as well as from the side effects caused from exogenous damage caused by food or drugs.

In particular, it has been found that chewable compositions prepared by using a fixed combination of hyaluronic acid, chondroitin sulfate and aluminum hydroxide, in admixture with a pharmaceutical carrier, allow the treatment of gastric pathologies such as upset stomach, heartburn, acid indigestion, gastritis, peptic ulcer, dyspepsia, gastric ulcer, gastroesophageal reflux; pathological hypersecretory conditions such as Zollinger Ellison Syndrome and also when a gastric epithelial damage is due to adverse effects of drugs. The presence of hyaluronic acid in the chewable compositions of the present invention allows, in respect of the treatment with compositions only containing chondroitin sulfate and aluminum hydroxide, an acceleration of the cicatrization of the damaged tissue much higher than expected. Unless otherwise specified, in the present description the percentages are expressed by weight, with respect to the total weight of the composition..

### DETAILED DECRIPTION

Thus, it is an aspect of the present invention to provide the use of a fixed combination of hyaluronic acid, chondroitin sulfate and aluminum hydroxide for the preparation of medicaments for the prevention or for the treatment of gastric disorders, especially for the treatment of gastritis or of gastric endothelial damages caused in particular by adverse effects of drugs. Said medicaments are compositions which may be pharmaceutical or medical device compositions wherein the fixed combination is in admixture with a pharmaceutical carrier or excipient.

More particularly, the invention provides the use of a fixed combination of hyaluronic acid, chondroitin sulfate and aluminum hydroxide for the preparation of chewable compositions for the prevention or for the treatment of gastric disorders, especially for the treatment of gastritis or of gastric endothelial damages, including those caused in particular by adverse effects of drugs.

The expression "fixed combination" is as defined in the EMEA (European Medicines Agency) Guidelines, i.e. "the combination of active substances within a single pharmaceutical form of administration is a so called 'fixed combination' of medicinal product".

Specifically, the fixed combination for use as set forth above advantageously consists of from 5 mg to 100 mg, advantageously from 5 mg to 75 mg, of hyaluronic acid or of a pharmaceutically acceptable salt thereof; from 200 mg to 700 mg, advantageously of from 200 mg to 500 mg, of chondroitin sulfate or a pharmaceutically acceptable salt thereof; and from 150 to 325 mg, advantageously from 150 mg to 280 mg, of aluminum hydroxide.

Preferably, the fixed combination to be used as set forth above consists of from 5 mg to 50 mg, more particularly from 5 mg to 25 mg, of hyaluronic acid or of a pharmaceutically acceptable salt thereof; from 200 mg to 450 mg, more particularly from 300 to 450 mg, of chondroitin sulfate or of a pharmaceutically acceptable salt thereof; and from 150 to 250 mg of aluminum hydroxide.

Said use is particularly indicated for the preparation of chewable compositions wherein the fixed combination as illustrated above is in admixture with a pharmaceutical carrier.

According to another aspect, the invention provides a chewable composition comprising hyaluronic acid, condroitin sulfate and aluminum hydroxide in admixture with a pharmaceutical carrier.

Hyaluronic acid and chondroitin sulfate of the present formulation are used as pharmaceutically acceptable salts thereof, in particular as an alkaline metal salt, such as sodium or potassium, with a preference for sodium, or alkaline-earth metal salt such as the calcium salt.

According to the present invention, in each chewable unit form hyaluronic acid or a pharmaceutically acceptable salt thereof is present in an amount of from 5 mg to 100 mg; chondroitin sulfate or a pharmaceutically acceptable salt thereof is present in an amount of from 200 mg to 700 mg, advantageously of from 200 mg to 500 mg; and aluminum hydroxide is present in an amount of from 150 mg to 325 mg, advantageously from 150 mg to 280 mg.

More particularly, it is an object of the present invention to provide a chewable composition comprising sodium hyaluronate in an amount of from 5 to 50 mg; sodium chondroitin sulfate in an amount of from 200 to 450 mg; and aluminum hydroxide in an amount of from 150 to 250 mg, in admixture with a pharmaceutical carrier.

Preferably, said composition comprises from 5 to 25 mg of sodium hyaluronate and from 300 to 400 mg of sodium chondroitin sulfate, the amount of aluminum hydroxide being of from 150 to 200 mg.

Advantageous chewable compositions contain from 0.5% to 1.5% hyaluronic acid, from 30% to 40% chondroitin sulfate and from 15% to 20% aluminum hydroxide. The chewable compositions of the present invention may be in any form, manufactured according to known technology, which can be masticated and swallowed dispersed in saliva, such as tablets, gummy sweets.

Carriers for chewable tablets include for example starches, cellulose and derivatives thereof; lubricants such as talc, stearic acid or magnesium stearate; diluents such as talc, powdered cellulose, lactose, polyethylenglycol, starches such as maize or corn starch, mannitol; disaggregating agents such as microcrystalline cellulose or crospovidone; ligands such as methylcellulose, sodium carboxymethylcellulose; sweeteners, or flavoring agents such as natural or synthetic oils. Tablets may also comprise sustained release ingredients such as glyceryl behenate, for example the products marketed as Compritol^{®} 888 ATO and Compritol^{®} E ATO, stearoyl macrogolglycerides, for example the product known under the trade name Gelucire^{®}, or glyceryl palmitostearate, for example the product known under the trade name Precirol^{®} ATO 5.

The sweeteners comprised in the orally chewable tablets may be natural, optional reduced sugars such as sucrose, dextrose, xylitol, mannitol or sorbitol, or synthetic product such as sodium saccharine or aspartame.

Synthetic sweeteners may be present in a percentage of from 0.1 to 5% while the natural, optional reduced sugar may be present at a percentage of from 10% to 20%, preferably of from 15% to 20%.

The flavoring agents are pharmaceutically acceptable flavors and tastes of synthetic and natural oils, the latter extracted from plants, leaves, flowers, fruits and their combinations, such as cinnamon, peppermint, anise and citron leaves, bitter almond, citrus fruits, in particular orange and/or lemon, linden and grapefruit oils. Also chocolate, vanilla or eucalyptus flavor and essences of fruit, in particular apple, pear, peach, strawberry, cherry, apricot, orange, lemon and grapes may be advantageously used.

Flavoring agents, usually supported on a solid matrix, are generally present at a concentration of from 0.5 to 1.5%.

Beside the main components of the fixed combination, preferably of that comprising from 5 to 25 mg of sodium hyaluronate; from 300 to 400 mg of sodium chondroitin sulfate; and aluminum hydroxide in an amount of from 150 to 200 mg, advantageous compositions of the present invention may include an alkaline-earth metal carbonate such as calcium carbonate or magnesium carbonate or an alkaline-earth hydroxide such as magnesium hydroxide, or aluminum-magnesium hydroxide or glycyrrhizic acid as a salt thereof, such as its ammonium or potassium salt.

Advantageous chewable tablets may also comprise calcium carbonate or ammonium glycyrrhizate or a mixture thereof.

The chewable compositions of the present invention are prepared by known pharmaceutical technology methods.

Chewable tablets, which are broken in the mouth by mastication and dispersed in saliva for swallowing, may comprise sweetening and flavoring agents as listed above to adjust the desired taste. Suitable dispersing agents include polyvinyl pyrrolidone such as Crospovidone^{®}, calcium carbonate and sodium starch glycolate. Aluminum hydroxide also favors the dispersion. The respective amounts of the dispersing and flavoring agents may be adjusted to reach a good equilibrium of dispersion and taste. Disintegrating agents such as Croscarmellose^{®} may also be added in concentrations that can vary from 3 to 10%. Typical carriers assuring the integrity of the tablet are talc, magnesium stearate and microcrystalline cellulose.

In advantageous chewable tablets, the fixed combination of hyaluronic acid, chondroitin sulfate and aluminum hydroxide may be added with ammonium glycyrrhyzinate and dispersed in a conventional chewable base, for example comprising sucrose, lactose or mannitol, optionally in the presence of a sustained release ingredient such as glyceryl behenate. Pharmaceutically acceptable salts of glycyrrhyzinic acid, such as the disodium and dipotassium salts, and monoammonium glycyrrhyzinate, are commercially available.

Preferably, in the compositions of the present invention both hyaluronic acid and chondroitin sulfate are present as sodium, potassium or calcium salt, with a preference for sodium.

As mentioned above, the compositions of the present invention are used for the prevention or for the treatment of gastritis or of gastric or duodenal endothelial damages, such as erosions or ulcers.

The ability of the fixed combination of the present invention to combat gastric disorders results from preliminar clinical observations made in patients suffering from gastritis. In a clinical study, the chewable tablets of Example 1 below were compared to a commercial chewable tablet preparation containing 400 mg aluminum hydroxide and 400 mg magnesium hydroxide in a small group of patients (5 per groups). Patients were selected as adult male or female aging not over 75 years, caucasian origin, normal physical examination, vital signs and laboratory evaluations, suffering from clinically confirmed gastritis. The patients of the Group I (Reference Group) were treated with a tablet of the commercial prepration four times/day for one month and those of Group 2 (Test Group) with a table according to Example 1 below, four times/day for one month. All the patients completed the treatment and no adverse effects were recorded. In the Reference Group a decrease of 40 % of the recurrent pain was measured while in the Test Group the decrease of recurrent pain was measured as 60%.

The following examples illustrate the invention.

### EXAMPLE 1

Chewable oral tablet weighing 1.100 mg comprising 10 mg of sodium hyaluronate, 400 mg of sodium chondroitin sulfate and 200 mg of aluminum hydroxide

| Component | Amount (in mg) |
|---|---|
| Sodium hyaluronate | 10.00 |
| Sodium chondroitin sulfate | 400.00 |
| Aluminum hydroxide | 200.00 |
| Calcium carbonate | 100.00 |
| Monoammonium glycyrrhizinate | 5.00 |
| Sucrose | 360.00 |
| Glyceryl behenate (Compritol^{®} E ATO) | 15.00 |
| Silicon bioxide | 10.00 |

Amounts of I Kg of sodium hyaluronate, 40 Kg of sodium chondroitin sulfate and 20 Kg of aluminum hydroxide are used. A preliminary mixture of 0.5 Kg of monoammonium glycyrrhizinate with hyaluronic acid (1 Kg) and half of the amount of sucrose (18 Kg) is mixed for 5 minutes in a blender, then 40 Kg of sodium chondroitin sulfate, 20 Kg of aluminum hydroxide, 10 Kg of calcium carbonate, 18 Kg of sucrose, and 1 Kg of silicon bioxide are added by further mixing for 7 minutes. At the end, 1 Kg of glyceryl behenate (Compritol^{®} E ATO) is added and mixing is continued for 5 minutes. The mixture is finally compressed to afford 100,000 tablets.

### EXAMPLE 2

Using the same method described in example 1, but with 15 mg of hyaluronic acid, 500 mg of chondroitin sulphate and 150 mg of aluminium hydroxide, 1.450 mg tablets were produced.

| *Component* | *Amount (in mg)* |
|---|---|
| Sodium hyaluronate | 15.00 |
| Sodium chondroitin sulfate | 500.00 |
| Aluminum hydroxide | 150.00 |
| Calcium carbonate | 300.00 |
| Monoammonium glycyrrhizinate | 5.00 |
| Sucrose | 450.00 |
| Glyceryl behenate (Compritol^{®} E ATO) | 15.00 |
| Silicon bioxide | 15.00 |

### EXAMPLE 3

Using the same method described in example 1, but with 10 mg of hyaluronic acid, 600 mg of chondroitin sulphate and 200 mg of aluminium hydroxide, 1.545 mg tablets were produced.

| *Component* | *Amount (in mg)* |
|---|---|
| Sodium hyaluronate | 10.00 |
| Sodium chondroitin sulfate | 600.00 |
| Aluminum hydroxide | 250.00 |
| Calcium carbonate | 200.00 |
| Monoammonium glycyrrhizinate | 5.00 |
| Sucrose | 450.00 |
| Glyceryl behenate (Compritol^{®} E ATO) | 15.00 |
| Silicon bioxide | 15.00 |

## Claims

1. Use of a fixed combination of hyaluronic acid, chondroitin sulfate and aluminum hydroxide for the preparation of medicaments for the prevention or for the treatment of gastric disorders.

2. The use of claim 1, wherein said fixed combination consists of from 5 mg to 100 mg of hyaluronic acid or of a pharmaceutically acceptable salt thereof; from 200 mg to 700 mg of chondroitin sulfate or a pharmaceutically acceptable salt thereof; and from 150 to 325 mg of aluminum hydroxide.

3. The use of claim 2, wherein said fixed combination consists of from 5 mg to 25 mg of hyaluronic acid or of a pharmaceutically acceptable salt thereof; from 300 to 450 mg of chondroitin sulfate or of a pharmaceutically acceptable salt thereof; and from 150 to 250 mg of aluminum hydroxide.

4. The use of anyone of claims 1 to 3 wherein said gastric disorders are gastritis or gastric or duodenal endothelial damages.

5. The use of claim 1, wherein said medicament is a chewable composition.

6. A chewable composition comprising hyaluronic acid or a pharmaceutically acceptable salt thereof, in an amount of from 5 mg to 100 mg; chondroitin sulfate or a pharmaceutically acceptable salt thereof, in an amount of from 200 mg to 700 mg; and aluminum hydroxide, in an amount of from 150 mg to 325 mg, in admixture with a pharmaceutical carrier.

7. The composition of claim 6, wherein said pharmaceutically acceptable salt is selected from the group consisting of alkaline metal and alkaline-earth metal salts.

8. The composition of claim 6, wherein said salt is selected from the group consisting of sodium, potassium and calcium salts of both chondroitin sulfate and hyaluronic acid.

9. The composition of claim 6, wherein hyaluronic acid is present as sodium salt in an amount of from 5 mg to 50 mg, chondroitin sulfate is present as sodium salt in an amount of from 200 mg to 450 mg and aluminum hydroxide is present in an amount of from 150 mg to 250 mg.

10. The composition of claim 9, wherein said amount of sodium hyaluronate is from 5 mg to 25 mg and said amount of sodium chondroitin sulfate is from 300 mg to 400 mg.

11. A fixed combination of hyaluronic acid, chondroitin sulfate and aluminum hydroxide for its use in the prevention or for its use in the treatment of gastric disorders.

12. The fixed combination according to claim 11, wherein said combination is a chewable composition.

13. The chewable pharmaceutical composition according to any one of claims 6 to 10, for its use in the prevention or for its use in the treatment of gastric disorders.

14. The composition according to any one of claims 11 to 13, wherein said gastric disorders are gastritis or gastric or duodenal endothelial damages.

## Patentansprüche

1. Verwendung einer fixen Kombination von Hyaluronsäure, Chondroitinsulfat und Aluminiumhydroxid zur Herstellung eines Medikaments für die Vorbeugung oder die Behandlung von Magenstörungen.

2. Verwendung gemäß Anspruch 1, wobei die fixe Kombination aus 5 mg bis 100 mg Hyaluronsäure oder eines pharmazeutisch annehmbaren Salzes davon, 200 mg bis 700 mg Chondroitinsulfat oder einem pharmazeutisch annehmbaren Salz davon und 150 bis 325 mg Aluminiumhydroxid besteht.

3. Verwendung gemäß Anspruch 2, wobei die fixe Kombination aus 5 mg bis 25 mg Hyaluronsäure oder eines pharmazeutisch annehmbaren Salzes davon, 300 mg bis 450 mg Chondroitinsulfat oder einem pharmazeutisch annehmbaren Salz davon und 150 bis 250 mg Aluminiumhydroxid besteht.

4. Verwendung gemäß einem der Ansprüche 1 bis 3, wobei die Magenstörungen Gastritis oder Magen- oder Zwölffingerdarm-Endothelschädigungen sind.

5. Verwendung gemäß Anspruch 1, wobei das Medikament eine kaubare Zusammensetzung ist.

6. Kaubare Zusammensetzung, umfassend Hyaluronsäure oder ein pharmazeutisch annehmbares Salz davon in eine Menge von 5 mg bis 100 mg, Chondoritinsulfat oder ein pharmazeutisch annehmbares Salz davon in einer Menge von 200 mg bis 700 mg und Aluminiumhydroxid in einer Menge von 150 mg bis 325 mg, in Beimischung mit einem pharmazeutischen Träger.

7. Zusammensetzung gemäß Anspruch 6, wobei das pharmazeutisch annehmbare Salz aus der Gruppe, bestehend aus Alkalimetall- oder Erdalkalimetallsalzen, ausgewählt ist.

8. Zusammensetzung gemäß Anspruch 6, wobei das Salz aus der Gruppe, bestehend aus Natrium-, Kalium- und Calciumsalzen von sowohl Chondroitinsulfat als auch Hyaluronsäure, ausgewählt ist.

9. Zusammensetzung gemäß Anspruch 6, wobei die Hyaluronsäure als Natriumsalz in einer Menge von 5 mg bis 50 mg anwesend ist, Chondroitinsulfat als Natriumsalz in einer Menge von 200 mg bis 450 mg anwesend ist und Aluminiumhydroxid in einer Menge von 150 mg bis 250 mg anwesend ist.

10. Zusammensetzung gemäß Anspruch 9, wobei die Menge an Natriumhyaluronat 5 mg bis 25 mg und die Menge an Natriumchondroitinsulfat 300 mg bis 400 mg beträgt.

11. Fixe Zusammensetzung von Hyaluronsäure, Chondroitinsulfat und Aluminiumhydroxid zur Verwendung in der Vorbeugung oder zur Verwendung in der Behandlung von Magenstörungen.

12. Fixe Zusammensetzung gemäß Anspruch 11, wobei die Zusammensetzung eine kaubare Zusammensetzung ist.

13. Kaubare pharmazeutische Zusammensetzung gemäß einem der Ansprüche 6 bis 10 für die Verwendung in der Vorbeugung oder die Verwendung in der Behandlung von Magenstörungen.

14. Zusammensetzung gemäß einem der Ansprüche 11 bis 13, wobei die Magenstörungen Gastritis oder Magen- oder Zwölffingerdarm-Endothelschädigungen sind.

## Revendications

1. Utilisation d'une combinaison fixe d'acide hyaluronique, de sulfate de chondroïtine et d'hydroxyde d'aluminium pour la préparation de médicaments pour la prévention ou pour le traitement de troubles gastriques.

2. Utilisation selon la revendication 1, dans laquelle ladite combinaison fixe consiste en 5 mg à 100 mg d'acide hyaluronique ou en un sel pharmaceutiquement acceptable de celui-ci ; 200 mg à 700 mg de sulfate de chondroïtine ou un sel pharmaceutiquement acceptable de celui-ci ; et 150 à 325 mg d'hydroxyde d'aluminium.

3. Utilisation selon la revendication 2, dans laquelle ladite combinaison fixe consiste en 5 mg à 25 mg d'acide hyaluronique ou en un sel pharmaceutiquement acceptable de celui-ci ; 300 à 450 mg de sulfate de chondroïtine ou en un sel pharmaceutiquement acceptable de celui-ci ; et 150 à 250 mg d'hydroxyde d'aluminium.

4. Utilisation selon l'une quelconque des revendications 1 à 3, dans laquelle lesdits troubles gastriques sont une gastrite ou des dommages endothéliaux gastriques ou duodénaux.

5. Utilisation selon la revendication 1, dans laquelle ledit médicament est une composition à mâcher.

6. Composition à mâcher comprenant de l'acide hyaluronique ou un sel pharmaceutiquement acceptable de celui-ci, dans une quantité de 5 mg à 100 mg ; du sulfate de chondroïtine ou un sel pharmaceutiquement acceptable de celui-ci, dans une quantité de 200 mg à 700 mg ; et de l'hydroxyde d'aluminium, dans une quantité de 150 mg à 325 mg, en mélange avec un vecteur pharmaceutique.

7. Composition selon la revendication 6, dans laquelle ledit sel pharmaceutiquement acceptable est choisi dans le groupe consistant en les sels de métaux alcalins et de métaux alcalino-terreux.

8. Composition selon la revendication 6, dans laquelle ledit sel est choisi dans le groupe consistant en les sels de sodium, potassium et calcium à la fois de sulfate de chondroïtine et d'acide hyaluronique.

9. Composition selon la revendication 6, dans laquelle l'acide hyaluronique est présent sous forme de sel de sodium dans une quantité de 5 mg à 50 mg, le sulfate de chondroïtine est présent sous forme de sel de sodium dans une quantité de 200 mg à 450 mg et l'hydroxyde d'aluminium est présent dans une quantité de 150 mg à 250 mg.

10. Composition selon la revendication 9, dans laquelle ladite quantité d'hyaluronate de sodium est de 5 mg à 25 mg et ladite quantité de sulfate chondroïtine de sodium est de 300 à 400 mg.

11. Combinaison fixe d'acide hyaluronique, de sulfate de chondroïtine et d'hydroxyde d'aluminium pour son utilisation dans la prévention ou pour son utilisation dans le traitement de troubles gastriques.

12. Combinaison fixe selon la revendication 11, dans laquelle ladite combinaison est une composition à mâcher.

13. Composition pharmaceutique à mâcher selon l'une quelconque des revendications 6 à 10, pour son utilisation dans la prévention ou pour son utilisation dans le traitement de troubles gastriques.

14. Composition selon l'une quelconque des revendications 11 à 13, dans laquelle lesdits troubles gastriques sont une gastrite ou des dommages endothéliaux gastriques ou duodénaux.
